# DEMANDE DE BREVET EUROPEEN

(11) **EP 2 078 716 A1**
(43) Date de publication de la demande: **15.07.2009**
(21) Numéro de dépôt: 08291259.3
(22) Date de dépôt: 31.12.2008
(51) Int. Cl.: C07D 401/12, A61K 31/404

(54) **Nouveaux dérivés d'1H-indol-1-yl urée, leur procédé de préparation et les compositions pharmaceutiques qui les contiennent**

(30) Priorité: 04.01.2008 FR 0800043
(71) Demandeur: Les Laboratoires Servier, 92415 Courbevoie Cedex (FR); Centre National de la Recherche Scientifique (CNRS), 75794 Paris Cedex 16 (FR); Université de Paris-Sud, 91405 Orsay Cedex (FR)
(72) Inventeur: Brion, Jean-Daniel, 95320 Saint Leu La Foret (FR); Deyine, Abdallah, 76250 Deville-Les-Rouen (FR); Le Ridant, Alain, 92200 Neuilly sur Seine (FR); Harpey, Catherine, 75016 Paris (FR)

(57) **Abrégé**

Composés de formule (I) : 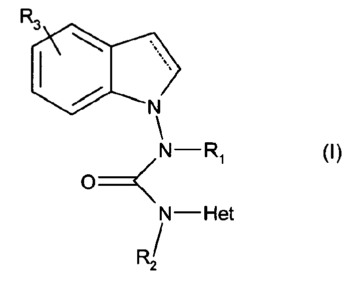 dans laquelle :
- R₁ et R₂, identiques ou différents, représentent un atome d'hydrogène ou un groupement alkyle (C₁-C₆) linéaire ou ramifié,
- R₃ représente un atome d'hydrogène ou d'halogène, un groupement alkyle (C₁-C₆) linéaire ou ramifié, ou un groupement alkoxy (C₁-C₆) linéaire ou ramifié,
- Het représente un groupement pyridyle, pyrimidyle ou pipéridyle, éventuellement substitués par un ou plusieurs groupements choisis parmi halogène, alkyle (C₁-C₆) linéaire ou ramifié, alkoxy (C₁-C₆) linéaire ou ramifié,
- ------ représente une liaison simple ou une liaison double,

leurs énantiomères et diastéréoisomères, ainsi que leurs sels d'addition à un acide ou à une base pharmaceutiquement acceptable,

Médicaments.

## Description

La présente invention concerne de nouveaux dérivés d'1*H*-indol-1-yl urée, leur procédé de préparation et les compositions pharmaceutiques qui les contiennent.

La littérature fournit de nombreux exemples de composés possédant une structure éburnane. C'est le cas notamment du brevet US 3 454 583 traitant de la vincamine ((3α,14β,16α)-(14,15-dihydro-14-hydroxy-éburnaménine-14-carboxylate de méthyle) et de ses dérivés pour leurs propriétés vasodilatatrices. Les demandes de brevets FR 2 433 528 et FR 2 381 048 présentent de nouveaux dérivés de 20,21-dinoréburnaménine et la demande EP 0 287 468, de nouveaux dérivés des 17-aza-20,21-dinoréburnaménine. La demande de brevet EP 0 658 557 décrit des dérivés de l'éburnane modifiés en position 14 et 15 du squelette éburnane. La demande de brevet EP 0 563 916 décrit des dérivés de 1*H*-indole-cyclohexanecarboxamide.

Les composés de la présente invention outre le fait qu'ils soient nouveaux, présentent des propriétés pharmacologiques très intéressantes. Ils se révèlent notamment être de puissants inducteurs de tyrosine hydroxylase, de manière sélective ou non.

Plus particulièrement, la présente invention concerne les composés de formule (I) : 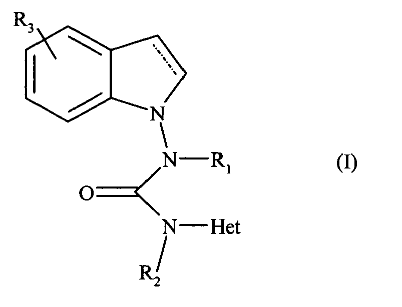 dans laquelle :
- R₁ et R₂, identiques ou différents, représentent un atome d'hydrogène ou un groupement alkyle (C₁-C₆) linéaire ou ramifié,
- R₃ représente un atome d'hydrogène ou d'halogène, un groupement alkyle (C₁-C₆) linéaire ou ramifié, ou un groupement alkoxy (C₁-C₆) linéaire ou ramifié,
- Het représente un groupement pyridyle, pyrimidyle, pipéridyle, 2-méthylpyridyle, 3-méthylpyridyle, 4-méthylpyridyle, phényle, benzyle, quinolinyle; pyridazinyle ou indolyle, chaque groupement étant éventuellement substitués par un ou plusieurs groupements choisis parmi halogène, alkyle (C₁-C₆) linéaire ou ramifié, alkoxy (C₁-C₆) linéaire ou ramifié,
- ----- représente une liaison simple ou une liaison double,
étant entendu que R₃ peut être greffé sur n'importe lequel des carbones du noyau indole/indoline le permettant,
leurs énantiomères et diastéréoisomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la *tert*butylamine, la lysine, etc...

Une variante avantageuse concerne les composés de formule (I) pour lesquels R₁, R₂ et R₃ représentent chacun un atome d'hydrogène.

Une variante encore plus avantageuse de l'invention concerne les composés de formule (I) pour lesquels Het est un groupe pyridinyle, pyrimidyle ou pipéridyle.

Un autre aspect particulier de l'invention concerne les composés de formule (I) pour lesquels Het est un groupe pyridinyle.

Encore plus particulièrement, l'invention concerne les composés de formule (I) qui sont :
- *N*-(1*H*-indol-1-yl)-*N*'-(3-pyridinyl)urée,
- *N*-(2,3-dihydro-1*H*-indol-1-yl)-*N*'-(3-pyridinyl)urée.

Les sels d'addition à un acide ou à une base pharmaceutiquement acceptables des composés préférés, font partie intégrante de l'invention.

La présente invention concerne également le procédé de préparation des composés de formule (I) caractérisé en ce que l'on utilise comme produit de départ un composé de formule (II): 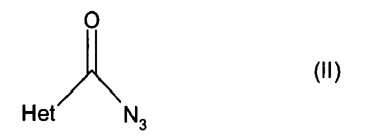 dans laquelle Het est tel que défini dans la formule (I),
composé (II), dont la décomposition thermique induit un dégagement de diazote, pour conduire à la formation d'un composé isocyanate de formule (III) que l'on peut isoler :

Het-N=C=O (III)

Le composé (III) est mis ensuite à réagir avec un composé de formule (IV) suivante : 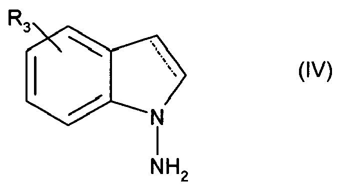 dans laquelle R₃ est tel que défini dans la formule (I),
pour conduire aux produits de l'invention de formule (I), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Les composés de formules (II) et (IV) sont soit des produits commerciaux, soit obtenus selon des méthodes classiques de la synthèse organique bien connues de l'homme du métier.

Les composés de formule (I) possèdent d'intéressantes propriétés pharmacologiques et notamment celle d'être de puissants inducteurs de tyrosine hydroxylase (TH). On sait que la tyrosine hydroxylase est une enzyme limitante qui contrôle particulièrement la synthèse des neurotransmetteurs dans les neurones catécholaminergiques et dopaminergiques centraux (Zhu M.-Y. et coll., Molecular Brain Research 133, (2005), 167-175). La vitesse de synthèse de ces neurotransmetteurs est notamment reliée à l'apparition de dysfonctions toniques du cerveau que sont de nombreuses pathologies comportementales chez l'homme, telles que l'anxiété, les psychoses, les dépressions, le stress, etc... (Schloss P. et coll., Pharmacology & Therapeutics 102, (2004), 47-60 ; Morilack D. A., et coll., International Journal of Neuropsychopharmacology 7, (2004), 193-218). Un deficit en noradrénaline et en dopamine dans le cortex préfrontal est la source des symptômes négatifs et cognitifs notamment dans la schizophrénie (Pira L. et coll., European Journal of Pharmacology 504, (2004), 61-64).

Grâce à leur capacité d'induction de tyrosine hydroxylase, les composés de l'invention trouvent donc leur utilisation thérapeutique dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.

La présente invention a également pour objet les compositions pharmaceutiques renfermant comme principe actif au moins un composé de formule (I), son énantiomère et diastéréoisomère ou un de ses sels d'addition à une base ou un acide pharmaceutiquement acceptable, seul ou en combinaison avec un ou plusieurs excipients ou véhicules inertes, non toxiques pharmaceutiquement acceptables.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par voie orale, rectale, intramusculaire ou parentérale.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires ou ovules, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 100 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrométriques usuelles (infrarouge, résonance magnétique nucléaire, spectrométrie de masse, ...).

Les points de fusion ont été déterminés sur appareil TOTTOLI (sans correction de colonne émergente). Lorsque le composé existe sous forme de sel, le point de fusion correspond à celui du produit salifié.

### PREPARATION 1 : nicotinoyl azide

À 2,4 ml d'acide chlorhydrique concentré (37 %) sont ajoutés à 0 °C 2 g de nicotinoylhydrazide, puis une solution de 2,02 g de nitrite de sodium dans 3,6 ml d'eau. Le milieu réactionnel est agité à 0°C pendant 30 minutes, puis traité par une solution saturée d'hydrogénocarbonate de sodium. Après extraction par de l'éther diéthylique (3 fois), la phase organique est lavée successivement par de l'eau et par une solution saturée de chlorure de sodium avant d'être séchée sur sulfate de magnésium. Après concentration sous pression réduite, on obtient le produit attendu (G. Papeo et coll., Synthesis, (2004), 2886).
*Infrarouge (*ν*_{cm-1}) :* 2178 (ν_{N3}); 1685 (ν_{CO}).

### PREPARATION 2: 2-pyridinecarbonyl azide

Le composé est obtenu suivant un procédé analogue à celui de la préparation 1 en remplaçant le nicotinoylhydrazide par le 2-pyridinecarbonylhydrazide.

### PREPARATION 3: isonicotinoyl azide

Le composé est obtenu suivant un procédé analogue à celui de la préparation 1 en remplaçant le nicotinoylhydrazide par l'isonicotinoylhydrazide.

### PREPARATION 4: 1-méthyl-3-pipéridinecarbonyl azide

Le composé est obtenu suivant un procédé analogue à celui de la préparation 1 en remplaçant le nicotinoylhydrazide par le 1-méthyl-3-pipéridinecarbonylhydrazide.

### PREPARATION 5: 1-méthyl-2-pipéridinecarbonyl azide

Le composé est obtenu suivant un procédé analogue à celui de la préparation 1 en remplaçant le nicotinoylhydrazide par le 1-méthyl-2-pipéridinecarbonylhydrazide.

### PREPARATION 6: 1-méthyl-4-pipéridinecarbonyl azide

Le composé est obtenu suivant un procédé analogue à celui de la préparation 1 en remplaçant le nicotinoylhydrazide par le 1-méthyl-4-pipéridinecarbonylhydrazide.

### PREPARATION 7: 5-méthoxy-2,3-dihydro-1H-indol-1-ylamine

### Stade A : 5-méthoxy-2,3-dihydro-1H-indole

0,991 g de 5-méthoxy-1*H*-indole est mis en solution dans 67 ml d'acide acétique glacial à température ambiante. 1,37 g de cyanoborohydrure de sodium est ajouté par portions. Après agitation à température ambiante pendant 1h 30, le mélange réactionnel est mis à sec; le résidu est traité par 50 ml d'eau et extrait 2 fois avec du dichlorométhane. La phase organique est lavée avec une solution saturée de NaHCO₃, puis avec une solution saturée de NaCl, séchée sur sulfate de magnésium et concentrée pour conduire au produit attendu.

### Stade B : 5-méthoxy-1-nitroso-2,3-dihydro-1H-indole

À une solution de 10 ml d'acide acétique à 50 % à 0 °C, est ajouté en une fois 0,499 g du composé du stade A précédent. À 0 °C, 0,232 g de nitrite de sodium en solution dans 5 ml d'eau est ajouté goutte à goutte. Une fois l'addition terminée, le mélange réactionnel est agité à 0 °C pendant 1h 30. Après addition d'eau, la phase aqueuse est extraite 3 fois par du dichlorométhane, les phases organiques réunies sont lavées par de l'eau et séchées sur sulfate de magnésium. L'évaporation à sec conduit au produit attendu qui est engagé dans la suite réactionnelle sans purification.

### Stade C : 5-méthoxy-2,3-dihydro-1H-indol-1-ylamine

À température ambiante, 0,353 g de 5-méthoxy-1-nitroso-2,3-dihydro-1*H*-indole est solubilisé dans 17 ml d'éthanol et 8,5 ml d'eau est additionnée. 1,42 g de zinc est ajouté, suivi de 1,89 g de carbonate d'ammonium. Après 1 h de vive agitation à température ambiante, la réduction est totale. Le zinc est éliminé par filtration sur célite et lavé par du méthanol. Après évaporation, le résidu est repris par de l'eau et extrait 3 fois avec du dichlorométhane. La phase organique est lavée avec une solution saturée de NaCl, séchée sur sulfate de magnésium et évaporée à sec pour livrer le produit attendu qui n'est pas purifiée pour l'étape suivante.

### PREPARATION 8: 1H-indol-1-ylcarbamate de phényle

3 g de 1*H*-indol-1-ylamine sont solubilisés dans 28 ml de dichlorométhane anhydre. 2,8 g de 4-(diméthylamino)pyridine sont ajoutés et le mélange réactionnel est refroidi à 0 °C. 2,85 ml de chloroformiate de phényle sont additionnés goutte à goutte et le mélange réactionnel est agité à 0 °C jusqu'à conversion totale en carbamate. Après évaporation des solvants, le résidu est repris dans un mélange pentane/dichlorométhane (50/50) et l'insoluble (chlorure de 4-(diméthylamino) pyridinium) est séparé par filtration. Le filtrat est concentré à sec et le résidu obtenu, solubilisé dans le dichlorométhane. La phase organique est lavée 3 fois par une solution d'acide chlorhydrique 0,1 M, puis par une solution saturée de NaCl. Après évaporation des solvants, le résidu obtenu est trituré dans un mélange pentane/éther diéthylique, puis séché sous vide phosphorique pour conduire au produit attendu.
*Point de fusion :* 144,5 °C.

### PREPARATION 9: Quinoléin-3-ylcarbamate de phényle

1,01 g de 3-aminoquinoléine sont solubilisés dans 20 ml de dichlorométhane anhydre. 1,12 g de 4-(diméthylamino)pyridine sont ajoutés et le mélange réactionnel est refroidi à 0 °C. 1,14 ml de chloroformiate de phényle sont additionnés goutte à goutte et le mélange réactionnel est agité à température ambiante pendant 1 h sous argon. Après évaporation des solvants, 50 ml d'eau sont ajoutés au résidu. Après 15 min, le carbamate forme un précipité blanc qui est, après filtration, séché pour conduire au produit attendu.
*Point de fusion :* 196 °C.

### PREPARATION 10: N-(1H-Indol-1-yl)-N-méthylcarbamate de phényle

48 mg d'hydrure de sodium sont ajoutés, sous atmosphère inerte, à une solution de 200 mg de *N*-(1*H*-indol-1-yl)carbamate de phényle dans 20 ml de tétrahydrofurane fraîchement distillé. Après agitation pendant dix minutes, 49 µl d'iodométhane sont ajouté. Le milieu réactionnel est agité sous atmosphère inerte pendant 4 heures. Après évaporation du tétrahydrofurane, 3 extractions par du dichlorométhane puis lavage par une solution saturée de NaCl, la phase organique est évaporée à sec. Le carbamate est purifié par flash chromatographie (acétate d'éthyle/cyclohexane : 1/9). Après évaporation des solvants, on obtient le produit attendu.
*Point de fusion :* 112 °C.

### EXEMPLE 1: N-(1H-indol-1-yl)-N'-(3-pyridinyl)urée

Une solution de 1,14 g du composé de la préparation 1 dans 38 ml de toluène est portée à reflux sous argon jusqu'à disparition totale de la matière première (2 h). À l'isocyanate de 3-pyridyle intermédiairement obtenu et refroidi à 0 °C, sont ajoutés 995 mg de 1*H-*indolamine dans 38 ml de dichlorométhane. L'agitation est poursuivie à température ambiante pendant 24 h. Le précipité formé est filtré et agité 12 h dans l'eau (10 ml). Après filtration et lavage par du pentane, le solide est repris dans un mélange de dichlorométhane / méthanol (90 / 10 ). Le solide, après filtration, est séché sous vide phosphorique pour conduire au produit attendu.
*Point de fusion :* 202,5 °C.

### EXEMPLE 2: N-(2,3-dihydro-1H-indol-1-yl)-N'-(3-pyridinyl)urée

Une solution de 942 mg du composé de la préparation 1 dans 30 ml de toluène est portée à reflux sous argon jusqu'à disparition totale de la matière première (1h 30). L'isocyanate de 3-pyridyle intermédiairement obtenu est refroidi à 0 °C et 824 mg de 1-indolinamine sont ajoutés en solution dans 30 ml de dichlorométhane. L'agitation est ensuite poursuivie à température ambiante pendant 15 h. Le précipité formé est filtré et lavé par de l'éther diéthylique ; il constitue un premier lot. Les eaux mères sont concentrées. Le résidu est repris dans le minimum de dichlorométhane, et de l'éther diéthylique est ajouté. Après filtration, le précipité formé constitue un deuxième lot qui est recristallisé dans l'acétate d'éthyle.
*Point de fusion :* 197 °C.

### EXEMPLE 3: N-(5-chloro-2,3-dihydro-1H-indol-1-yl)-N'-(3-pyridinyl)urée

Le composé est obtenu suivant un procédé analogue à celui de l'exemple 2 en remplaçant la 1-indolinamine par la 5-chloro-1-indolinamine.

### EXEMPLE 4: N-(5-méthoxy-1H-indol-1-yl)-N'-(3-pyridinyl)urée

Le composé est obtenu suivant un procédé analogue à celui de l'exemple 1 en remplaçant la 1*H*-indolamine par la 5-méthoxy-1*H*-indol-1-amine.
Une solution de 0,281 g du composé de la préparation 1 dans 9,25 ml de toluène est portée à reflux, sous argon, jusqu'à conversion totale en isocyanate (2 h). À cette solution, refroidie à 0 °C, est ajoutée 0,300 g de 5-méthoxy-1*H*-indol-1-ylamine solubilisée dans 9,25 ml de dichlorométhane. L'agitation est poursuivie à température ambiante pendant 24 h. Le précipité formé, après filtration, est solubilisé dans du dichlorométhane et la phase organique obtenue est lavée 3 fois avec de l'eau. Après séchage sur sulfate de magnésium puis évaporation à sec, le résidu est trituré avec du pentane et séché sous vide phosphorique pour conduire au produit attendu.
*Point de fusion :* 176 °C.

### EXEMPLE 5: N-(2,3-dihydro-1H-indol-1-yl)-N'-(2-pyridinyl)urée

Le composé est obtenu suivant un procédé analogue à celui de l'exemple 2 en remplaçant la préparation 1 par la préparation 2.

### EXEMPLE 6: N-(1H-indol-1-yl)-N'-(4-pyridinyl)urée

Le composé est obtenu suivant un procédé analogue à celui de l'exemple 1 en remplaçant la préparation 1 par la préparation 3.
Une solution de 0,305 g du composé de la préparation 3 dans 8,8 ml de toluène est portée à reflux sous argon jusqu'à conversion totale en isocyanate (2 h). Au milieu réactionnel refroidi à 0 °C, est ajoutée 0,265 g de 1*H*-indol-1-ylamine dans 8,8 ml de dichlorométhane. L'agitation est poursuivie à température ambiante pendant 5 jours. Le précipité formé est, après filtration, purifié par chromatographie sur colonne de silice (acétate d'éthyle/cyclohexane : 60/40). Après évaporation des solvants, le résidu est lavé par du pentane, séché sous vide à 70 °C, puis sous vide phosphorique pendant 12 h à température ambiante pour conduire au produit attendu.
*Point de fusion :* 138 °C.

### EXEMPLE 7: N-(1H-indol-1-yl)-N'-(l-méthyl-3-pipéridinyl)urée

Le composé est obtenu suivant un procédé analogue à celui de l'exemple 1 en remplaçant la préparation 1 par la préparation 4.

### EXEMPLE 8: N-(1H-indol-1-yl)-N'-(1-méthyl-2-pipéridinyl)urée

Le composé est obtenu suivant un procédé analogue à celui de l'exemple 1 en remplaçant la préparation 1 par la préparation 5.

### EXEMPLE 9: N-(1H-indol-1-yl)-N'-(1-méthyl-4-pipéridinyl)urée

Le composé est obtenu suivant un procédé analogue à celui de l'exemple 1 en remplaçant la préparation 1 par la préparation 6.

### EXEMPLE 10: N-(2,3-dihydro-1H-indol-1-yl)-N'-(1-méthyl-3-pipéridinyl)urée

Le composé est obtenu suivant un procédé analogue à celui de l'exemple 2 en remplaçant la préparation 1 par la préparation 4.

### EXEMPLE 11: N-(5-méthyl-1H-indol-1-yl)-N'-(3-pyridinyl)urée

Une solution de 0,598 g du composé de la préparation 1 dans 20 ml de toluène est portée à reflux sous argon jusqu'à conversion totale en isocyanate (1h 30). Au milieu réactionnel refroidi à 0 °C, est ajoutée 0,620 mg de 5-méthyl-1*H*-indol-1-ylamine dans 5 ml de dichlorométhane. L'agitation est ensuite poursuivie à température ambiante pendant 24 h. Le précipité formé est, après filtration, lavé par du dichlorométhane puis du pentane. Il est repris dans l'éthanol chaud ; les cristaux obtenus sont séchés sous vide phosphorique pendant 6 h à 70 °C pour conduire au produit attendu.
*Point de fusion :* 197,5 °C.

### EXEMPLE 12: N-(5-méthoxy-2,3-dihydro-1H-indol-1-yl)-N'-(3-pyridinyl)urée

Une solution de 0,270 g du composé de la préparation 1 dans 9 ml de toluène est portée à reflux sous argon jusqu'à conversion totale en isocyanate (1 h). Au milieu réactionnel refroidi à 0 °C est ajoutée 0,300 g du composé de la préparation 7 dans 5 ml de dichlorométhane. L'agitation est ensuite poursuivie à température ambiante et après 24 h, la conversion en urée est totale. Le précipité formé est, après filtration, lavé par du dichlorométhane, puis du pentane. Le produit brut est purifié par chromatographie sur plaques préparatives (acétate d'éthyle/méthanol : 95/5). Après filtration sur Acrodisc^{®} GHP 0,45 µm du produit élué, un lavage à l'éthanol chaud fournit des cristaux du produit attendu.
*Point de fusion :* 177,5 °C.

### EXEMPLE 13: N-(5-chloro-1H-indol-1-yl)-N'-(4-pyridinyl)urée

Une solution de 0,500 g du composé de la préparation 3 dans 17 ml de toluène est portée à reflux sous argon, jusqu'à conversion totale (1h30) en isocyanate. Au milieu réactionnel refroidi à 0 °C, est ajoutée 0,562 g de 5-chloro-1*H*-indol-1-ylamine dans 17 ml de dichlorométhane. L'agitation est ensuite poursuivie à température ambiante pendant 24 h. Le précipité formé est, après filtration, purifié par chromatographie sur colonne de silice (acétate d'éthyle/cyclohexane/méthanol : 60/40/5). Après évaporation partielle des solvants et filtration sur Acrodisc^{®} PSF 0.45 µm, le résidu est lavé par du pentane, séché sous vide phosphorique à 70 °C pendant 6 h pour conduire au produit attendu.
*Point de fusion :* 267,5 °C.

### EXEMPLE 14: N-(5-chloro-1H-indol-1-yl)-N'-(2-pyridinyl)urée

2,45 g de carbonyldiimidazole est solubilisé dans 12 ml de tétrahydrofurane à 0 °C. 1,023 g de 5-chloro-1*H*-indol-1-ylamine solubilisée dans 50 ml de tétrahydrofurane, est ajoutée goutte à goutte. Le mélange réactionnel est agité à 0 °C jusqu'à conversion totale en imidazolide (48 h). Après élimination du solvant, le résidu est dissous dans du dichlorométhane; la phase organique obtenue est lavée deux fois à l'eau, une fois avec une solution saturée de NaCl et séchée sur sulfate de magnésium. L'évaporation du solvant fournit l'imidazolide sous forme d'une huile visqueuse instable à température ambiante. À une solution de 0,510 g d'imidazolide dans 5 ml de dichlorométhane, est ajoutée goutte à goutte sous azote, 0,500 g de 2-aminopyridine. Le mélange réactionnel est agité à température ambiante. Après 24 h, un précipité est observé et l'agitation est poursuivie. Après 72 h, de l'éther diéthylique est ajouté et le solide est séparé par filtration. Le solide et le filtrat sont purifiés séparément par flash chromatographie et les fractions contenant le produit attendu sont rassemblées.
*Point de fusion :* >320 °C (décomposition).

### EXEMPLE 15: N-(1H-indol-1-yl)-N'-(2-pyrimidinyl)urée

0,408 g du composé de la préparation 8 et 0,175 g de 2-aminopyrimidine sont chauffés à reflux du toluène jusqu'à conversion totale en urée (72 h). Le précipité obtenu est séparé par filtration, puis lavé par un mélange méthanol/éthanol chaud pour conduire au produit attendu.
*Point de fusion :* 263 °C.

### EXEMPLE 16: N-(1H-indol-1-yl)-N'-(2-pyridinyl)urée

0,500 g du composé de la préparation 8 et 0,379 g de 2-aminopyridine sont chauffés à 70 °C dans 10 ml de toluène anhydre pendant environ 72 h. Le précipité est séparé par filtration, lavé avec du pentane puis séché pour conduire au produit attendu.

### EXEMPLE 17: N-(1H-indol-1-yl)-N'-(phényl)urée

À 0 °C, 0,300 g de 1*H*-indol-1-ylamine sont solubilisés dans du 11 ml de dichlorométhane anhydre. 245 µl d'isocyanate de phényle commercial en solution dans 11 ml du dichlorométhane sont ajoutés goutte à goutte. Le mélange réactionnel est laissé revenir à température ambiante pendant 12 h. Après 4 jours à température ambiante, le précipité observé est, après filtration, lavé par du pentane, puis séché sous vide phosphorique à 70 °C pour conduire au produit attendu.
*Point de fusion :* 243 °C.

### EXEMPLE 18: N-(1H-indol-1-yl)-N'-(benzyl)urée

À 0 °C, 0,308 g de 1*H*-indol-1-ylamine sont solubilisés dans 11 ml de dichlorométhane anhydre. 300 µl d'isocyanate de benzyle commercial solubilisé dans 11 ml de dichlorométhane sont ajoutés goutte à goutte. Le mélange réactionnel est laissé revenir à température ambiante pendant 12 h. Après 4 jours à température ambiante, le précipité obtenu est, après filtration, lavé par du pentane, puis séché sous vide phosphorique à 70 °C pour conduire au produit attendu.
*Point de fusion :* 204 °C.

### EXEMPLE 19: N-(5-chloro-1H-indol-1-yl)-N'-(benzyl)urée

À 0 °C, 0,301 g de 5-chloro-1*H*-indol-1-ylamine sont solubilisés dans 8 ml de dichlorométhane anhydre. 245 µl d'isocyanate de benzyle commercial sont ajoutés goutte à goutte. Le mélange réactionnel est laissé revenir à température ambiante pendant 12 h. Après 5 jours à température ambiante, le précipité observé est, après filtration, lavé par du pentane puis séché sous vide phosphorique à 70 °C pour conduire au produit attendu.
*Point de fusion :* 218 °C.

### EXEMPLE 20: N-(5-chloro-1H-indol-1-yl)-N'-(phényl)urée

À 0 °C, 0,300 g de 5-chloro-1*H*-indol-1-ylamine sont solubilisés dans 8 ml de dichlorométhane anhydre. 195 µl d'isocyanate de phényle commercial sont ajoutés goutte à goutte. Le mélange réactionnel est laissé revenir à température ambiante pendant 12 h. Après 5 jours à température ambiante, le précipité obtenu est, après filtration, lavé par du pentane, puis séché sous vide phosphorique à 70 °C pour conduire au produit attendu.
*Point de fusion :* 247,5 °C.

### EXEMPLE 21: N-(1H-indol-1-yl)-N'-(3-pyridinylméthyl)urée

2,45 g de carbonyldiimidazole sont solubilisés dans 12 ml de tétrahydrofurane à 0 °C. 1,023 g de 1*H*-indol-1-ylamine en solution dans 50 ml de tétrahydrofurane sont ajoutés goutte à goutte. Le mélange réactionnel est agité à 0 °C jusqu'à conversion totale en imidazolide (48 h). Après évaporation du solvant, le résidu est dissous dans du dichlorométhane; la phase organique obtenue est lavée deux fois avec de l'eau, une fois avec une solution saturée de NaCl et séchée sur sulfate de magnésium. L'élimination du solvant fournit le *N*-(1*H*-indol-1-yl)-1*H*-imidazole-1-carboxamide. À une solution de 0,600 g de *N*-(1*H*-indol-1-yl)-1*H*-imidazole-1-carboxamide dans 2,5 ml de dichlorométhane anhydre, sont ajoutés 135 µl de triéthylamine. 135 µl de 3-(aminométhyl)pyridine solubilisés dans 2,5 ml de dichlorométhane anhydre, sont ajoutés goutte à goutte, puis le mélange réactionnel est agité à température ambiante pendant 4 jours. Après élimination des solvants, le résidu obtenu est purifié par flash chromatographie (acétate d'éthyle/cyclohexane : 10/40, puis 50/50). Après évaporation des solvants, le solide obtenu est lavé par de l'éthanol chaud, puis séché après filtration pour conduire au produit attendu.
*Point de fusion :* 159,5 °C.

### EXEMPLE 22: N,N'-bis(5-chloro-1H-indol-1-yl)-urée

0,310 g de carbonyldiimidazole sont solubilisés dans 5 ml de tétrahydrofurane. 0,501 g de 5-chloro-1*H*-indol-1-ylamine sont ajoutés et le mélange réactionnel est agité à température ambiante. Après 12 h, un précipité est observé ; le milieu réactionnel est ensuite chauffé au reflux du tétrahydrofurane pendant 6 h. Après filtration du précipité, lavage par du dichlorométhane puis du pentane, le solide est séché sous vide pour conduire au produit attendu.
*Point de fusion :* >300 °C.

### EXEMPLE 23: N,N'-bis(1H-indol-1-yl)-urée

Le composé est obtenu suivant un procédé analogue à celui de l'exemple 22 en remplaçant la 5-chloro-1*H*-indol-1-ylamine par la 1*H*-indol-1-ylamine.
*Point de fusion :* >347 °C.

### EXEMPLE 24: N-(1H-indol-1-yl)-N'-(2-pyridinylméthyl)urée

0,715 g de carbonyldiimidazole sont solubilisés dans 5 ml de tétrahydrofurane à 0 °C. 0,258 g de 1*H*-indol-1-ylamine en solution dans 25 ml de tétrahydrofurane sont ajoutés goutte à goutte. Le mélange réactionnel est agité à 0 °C pendant 4 h, puis laissé à température ambiante. 1,2 ml de 2- (méthylamino)pyridine sont ajoutés au mélange réactionnel; après 48 h d'agitation à température ambiante, le solide obtenu est, après filtration, lavé par de l'éther diéthylique. Le filtrat est concentré sec et purifié par flash chromatographie (acétate d'éthyle/cyclohexane : 30/70 puis 55/45). Après évaporation des solvants, le solide obtenu est lavé par du pentane, puis séché après filtration pour conduire au produit attendu.
*Point de fusion :* 163 °C.

### EXEMPLE 25: N-(1H-indol-1-yl)-N'-(3-quinolinyl)urée

1,30 g du composé de la préparation 9 et 0,500 g de N-amino-1*H*-indole sont chauffés à reflux de l'acétonitrile sous argon pendant 20 h. Le solvant est alors évaporé sous vide et le résidu est repris avec 100 ml d'une solution aqueuse de carbonate de sodium, puis extrait avec 3 x 30 ml de dichlorométhane. La phase organique est séchée sur sulfate de sodium, filtrée et concentrée sous pression réduite pour fournir 1,50 g de produit brut. La recristallisation de l'urée dans l'éthanol permet de conduire au produit attendu.
*Point de fusion :* 235 °C.

### EXEMPLE 26: N-(1H-indol-1-yl)-N'-(2-pyridazinyl)urée

0,409 g de 1*H*-indol-1-ylcarbamate de phényle et 0,152 g de 2-aminopyridazine sont chauffés à reflux du toluène jusqu'à conversion totale en urée (72 h). Le précipité obtenu est, après filtration, lavé par du toluène chaud, puis par de l'acétone plusieurs fois. Le solide est enfin lavé par de l'éthanol à chaud, puis après filtration, séché pour conduire au produit attendu.
*Point de fusion :* 220 °C.

### EXEMPLE 27: N-(1H-indol-1-yl)-N'-(4-pyridinyiméthyl)urée

0,499 g de 1*H*-indol-1-ylcarbamate de phényle et 0,45 ml de 4-(aminométhyl)pyridine sont chauffés à 70 °C dans 10 ml de toluène anhydre pendant environ 36 h. Le précipité est séparé par filtration, puis lavé par du pentane chaud. Après reprise par de l'éthanol chaud et filtration, il est séché sous vide phosphorique à 70 °C pour conduire au produit attendu.
*Point de fusion :* 182 °C.

### EXEMPLE 28: N-(1H-indol-1-yl)-N'-(6-quinolinyl)urée

0,502 g de 1*H*-indol-1-ylcarbamate de phényle et 0,577 g de 6-aminoquinoléine sont chauffés à 70 °C dans 10 ml de toluène anhydre pendant environ 72 h. Le précipité est séparé par filtration, lavé par du pentane, puis séché sous vide phosphorique à 70 °C pour conduire au produit attendu.
*Point de fusion :* 249 °C.

### EXEMPLE 29: N-(1H-indol-1-yl)-N'-(5-quinolinyl)urée

0,405 g de 1*H*-indol-1-ylcarbamate de phényle et 0,459 g de 5-aminoquinoléine sont chauffés à 70 °C dans 10 ml de toluène anhydre pendant environ 36 h. Le précipité est séparé par filtration, lavé avec de l'acétone chaud, puis séché sous vide phosphorique à 70 °C pour conduire au produit attendu.
*Point de fusion :* 288 °C.

### EXEMPLE 30: N-(6-chloro-3-pyridazinyl)-N'-(1H-indol-1-yl)urée

0,498 g de 1*H*-indol-1-ylcarbamate de phényle et 1,32 g de 3-amino-6-chloropyridazine sont chauffés à 70 °C dans 10 ml de toluène anhydre pendant 10 jours. Le précipité, après filtration, est solubilisé dans du dichlorométhane. La phase organique est lavée 3 fois avec une solution d'acide chlorhydrique 0,2 M, puis par une solution saturée de NaCl. Après séchage sur sulfate de magnésium, les phases organiques sont concentrées à sec et le résidu obtenu est repris par de l'éther diéthylique, puis par du dichlorométhane. L'urée est séchée sous vide phosphorique à 70 °C pour conduire au produit attendu.
*Spectrométrie de masse (ESI, m*/*z):* 286 (M-1) ; 288 (M+1).

### EXEMPLE 31: N-(1H-indol-1-yl)-N'-(8-quinolinyl)urée

À une solution de 0,790 g de 8-aminoquinoléine dans 5,5 ml de toluène, refroidie à 0 °C, sont additionnés goutte à goutte 4,5 ml d'une solution 1 M de triméthylaluminium. Après 10 min d'agitation à 0 °C, le mélange réactionnel est laissé revenir à température ambiante et agité 1h à cette température. 0,390 g de 1*H*-indol-1-ylcarbamate de phényle sont mis en suspension dans 5,5 ml de toluène à 0 °C; à cette suspension, est ajouté le complexe aluminique par l'intermédiaire d'une canule. Après retour à température ambiante, le mélange réactionnel est chauffé à 65 °C jusqu'à conversion totale en urée (1h 30). Le milieu réactionnel est hydrolysé par de l'eau et le toluène, éliminé par évaporation sous vide. Le résidu, repris par de l'eau, est extrait par du dichlorométhane. La phase organique est lavée 3 fois avec une solution d'acide chlorhydrique 0,5 M, puis par une solution saturée de NaCl, puis séchée sur sulfate de magnésium. Après élimination des solvants, le résidu est recristallisé dans un mélange pentane/éther diéthylique (90/10). Après filtration, les cristaux obtenus sont séchés sous vide phosphorique à 70 °C pour conduire au produit attendu.
*Point de fusion :* 201 °C.

### EXEMPLE 32: N-(1H-indol-1-yl)-1-méthyl-3-(3-pyridinyl)urée

À une solution de 100 mg de N-(1*H*-indol-1-yl)-N-méthylcarbamate de phényle dans 20 ml de diméthylformamide anhydre, sont ajoutées 54 mg de 3-aminopyridine et 220 mg de (4-diméthylamino)pyridine. La solution est agitée pendant trois jours au reflux du diméthylformamide. Après 3 extractions par de l'acétate d'éthyle, puis lavage par une solution saturée de NaCl, la phase organique est évaporée à sec. L'urée est purifiée par flash chromatographie (acétate d'éthyle/cyclohexane : 3/7) pour conduire au produit attendu.
*Spectrométrie de masse (ESI, m*/*z):* 289,1 (M+23).

### EXEMPLE 33: N-(1H-Indol-1-yl)-3-méthyl-3-(3-pyridinyll)urée

À une solution de 313 mg de (1*H*-indol-1-yl)carbamate de phényle dans 10 ml de toluène anhydre, est ajoutée 135 g de 3-(méthylamino)pyridine. La solution est agitée sous argon pendant deux jours au reflux du toluène puis abandonnée à température ambiante pendant deux jours. Le précipité formé est filtré pour donner le produit attendu.
*Point de fusion :* 168 °C.

### EXEMPLE 34: N-(1H-Indol-1-yl)-1,3-diméthyl-3-(3-pyridinyll)urée

80 mg d'hydrure de sodium sont additionnés, sous atmosphère inerte, à une solution de 252 mg de 1-(1*H*-indol-1-yl)-3-(pyridin-3-yl)urée dans 20 ml de tétrahydrofurane fraîchement distillé. Après agitation pendant dix minutes, 124 µl d'iodométhane sont ajoutés. Le milieu réactionnel est agité sous atmosphère inerte pendant 2 jours. Après évaporation du tétrahydrofurane, 3 extractions par du dichlorométhane puis, lavage par une solution saturée de NaCl, la phase organique est évaporée à sec. L'urée est purifiée par flash chromatographie (dichlorométhane/méthanol : 95/5) pour donner le produit attendu.
*Point de fusion :* 99 °C.

### ETUDE PHARMACOLOGIOUE DES DERIVES DE L'INVENTION

### EXEMPLE A : Induction de tyrosine hydroxylase

Il est recherché, parmi les dérivés, ceux qui sont capables d'induire une augmentation de la protéine tyrosine hydroxylase (TH) dans le cerveau de la souris Balb/C, au niveau du locus coeruleus (LC).
Les animaux utilisés sont des souris mâles de la lignée pure Balb/C
(Charles River Laboratories), âgées de 7 à 8 semaines au moment du traitement.

Les souris reçoivent une injection unique par voie intra péritonéale du composé à tester, dissous dans une solution d'HCl 0.04 M (contrôle correspondant : HCl 0.004 M) si le composé est suffisamment soluble, ou dans l'huile d'olive 90 % / DMSO 10 % (contrôle correspondant : huile d'olive 90 % / DMSO 10 %) pour les composés insolubles en milieu aqueux.

Trois jours après l'injection de chaque molécule, tous les animaux sont sacrifiés par décapitation. Les cerveaux extraits sont ensuite congelés dans de l'azote liquide et conservés à -80° C.

Des coupes coronales de 8 microns d'épaisseur sont pratiquées le long de l'axe postéro-antérieur du LC et fixées. Les coupes sont transférées sur membranes Immobilon-P. La TH est mesurée par immunodétection et analyse d'image.

### Résultats :

Les résultats d'induction de la TH au niveau du LC sont présentés dans le tableau I suivant.

**TABLEAU I**

| **Mesure de la quantité de TH au niveau des différentes coupes du LC numérotées de 1 à 8 dans le sens antérieur vers postérieur, après administration i.p. (20 mg/kg)** | | | | | | | | |
|---|---|---|---|---|---|---|---|---|
| *Les résultats sont exprimés en % par rapport à la valeur moyenne du groupe témoin¹* | | | | | | | | |
| % | 1 | 2 | 3 | 4 | 5 | 6 | 7 | 8 |
| Exemple 1 | 66 | 85 | 74 | 64 | 45 | 40 | 8 | 2 |
| Exemple 2 | 63 | 77 | 64 | 57 | 34 | 15 | 8 | 2 |
| Exemple 6 | 65 | 81 | 59 | 33 | 20 | 13 | 8 | 2 |
| Exemple 24 | 74 | 78 | 68 | 45 | 29 | 19 | 5 | 7 |

| | | | | | | | | |
|---|---|---|---|---|---|---|---|---|
| ¹animaux traités par le même véhicule | | | | | | | | |

### EXEMPLE B : Prédiction de stabilité métabolique

La prédiction de stabilité métabolique est testée par incubation des dérivés à la concentration de 10⁻⁷ M en présence de microsomes hépatiques de souris, rat ou d'homme (0,33 mg prot/ml). Après addition de NADPH (nicotinamide adénine dinucléotide phosphate, forme réduite), des prélèvements sont réalisés à 0, 5, 15, 30 et 60 minutes. La réaction enzymatique est arrêtée avec le méthanol (V/V). La protéine est précipitée par centrifugation et le surnageant analysé par LC-MS-MS.
La bonne stabilité métabolique de ces dérivés permet d'envisager un traitement per os.

**TABLEAU II**

| **% de prédiction de stabilité métabolique sur microsomes hépatiques** | | | |
|---|---|---|---|
| | Souris | Rat | Homme |
| Exemple 1 | 78 | 78 | 72 |
| Exemple 2 | 85 | 55 | 98 |
| Exemple 6 | 77 | 73 | 77 |
| Exemple 13 | 84 | 89 | 82 |
| Exemple 24 | 72 | 69 | 67 |

Les composés qui présentent une bonne prédiction de stabilité métabolique chez l'homme présentent l'avantage de pouvoir être administrés par voie orale (entre 67 et 82 %).

### EXEMPLE C : Prédiction de passage de la barrière hémato-encéphalique (BHE)

Les substances sont incubées à 20 µM dans le compartiment supérieur d'une double cuve séparé du compartiment inférieur soit par un filtre de polycarbonate seul, soit par le même filtre recouvert de cellules endotahéliales confluentes de capillaires bovins. L'évaluation de la cinétique de perméabilité est réalisée par quantification LC-MS-MS de la substance inchangée dans le compartiment inférieur après 10, 20, 30, 40 et 60 minutes.
Les dérivés testés présentent un passage généralement élevé de la BHE favorisant la cible neurologique. Les résultats sont rendus sous forme de classes : élevé, intermédiaire, faible. Ainsi, l'exemple 1 présente un passage élevé de la BHE.

### EXEMPLE D : Augmentation de la phosphorylation de ERK dans les structures cérébrales spécifiques et responsables de l'activité

ERK est une kinase qui, sous sa forme phosphorylée p-ERK, active certaines protéines dont la tyrosine hydroxylase. Celle-ci, à la fois augmentée, et activée par phosphorylation, favorise la synthèse de la dopamine et de la noradrénaline dont la carence est à l'origine de pathologies neurologiques et psychiatriques comme la maladie de Parkinson, les formes déficitaires de schizophrénie, la dépression ...

On sait que l'administration d'un inhibiteur de la phosphorylation de ERK entraîne un comportement dépressif chez la souris (Duman C.H. et coll., Biological Psychiatry 61, (2007), 661-670).
L'exposition de rat à un stress s'accompagne de la réduction de la phosphorylation de ERK (Yang C.-H. et coll, The Journal of Neuroscience 24(49), (2004), 11029-11034).
ERK est un acteur crucial de la plasticité synaptique et neuronale nécessaire à l'acquisition et au bon fonctionnement de la mémorisation. L'inhibition de sa phosphorylation entraîne des troubles de la cognition (Adams J.P. et coll., Annual Review of Pharmacology and Toxicology 42, (2002), 135-163).
L'administration de phencyclidine, qui reproduit les symptômes de la schizophrénie chez l'homme comme chez l'animal, inhibe la phosphorylation de ERK *in vitro* comme *in vivo* (Enomoto T. et coll, Molecular pharmacology 68, (2005), 1765-1774).

Le rôle du composé de l'exemple 1 sur la phosphorylation de ERK a été mis en évidence *in vitro* et sur des structures *ex vivo* :

### In vitro

### 1. Méthode

Différentes formes de ERK-recombinant non phosphorylé (ERK1 et ERK2) et phosphorylé (p-ERK1 et p-ERK2) sont fixées séparément sur les "puces" et étudiées dans le système Biacore^{®}. L'interaction directe du composé de l'exemple 1 à différentes concentrations est étudiée.

### 2. Résultats

Le composé de l'exemple 1 se lie fortement à ERK1 et ERK2 de manière dose-dépendante. Cette liaison est inhibée si on ajoute l'ERK phosphorylé correspondant dans le milieu.

### Ex vivo sur tissus cérébraux de cochon

### 1. Méthode

Les différentes structures cérébrales sont isolées, traitées et mises en suspension dans un milieu sans phosphate, après inactivation des phosphatases des tissus. Les tissus sont débarrassés de leur propre ERK et p-ERK et mis en présence des formes spécifiques recombinantes de ERK1, ERK2, p-ERK1 et p-ERK2.
Les capacités de phosphorylation sont étudiées en présence ou non du composé de l'exemple 1 par des anticorps permettant d'évaluer ERK1 et ERK2 non phosphorylés et phosphorylés.

### 2. Résultats

Le composé de l'exemple 1 à la concentration de 3.10⁻⁹ M augmente la phosphorylation de ERK1 et ERK2 de manière préférentielle dans l'hippocampe, le cortex préfrontal, de ERK2 également dans le striatum.

### EXEMPLE E : Composition pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3g |

## Revendications

1. Composés de formule (I) : 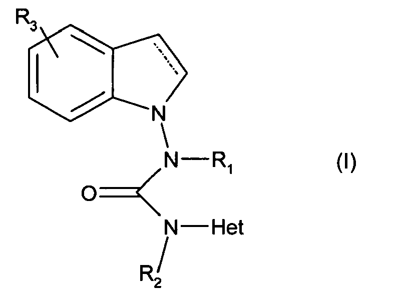 dans laquelle :
- R₁ et R₂, identiques ou différents, représentent un atome d'hydrogène ou un groupement alkyle (C₁-C₆) linéaire ou ramifié,
- R₃ représente un atome d'hydrogène ou d'halogène, un groupement alkyle (C₁-C₆) linéaire ou ramifié, ou un groupement alkoxy (C₁-C₆) linéaire ou ramifié,
- Het représente un groupement pyridyle, pyrimidyle, pipéridyle, 2-méthylpyridyle, 3-méthylpyridyle, 4-méthylpyridyle, phényle, benzyle, quinolinyle; pyridazinyle ou indolyle, chaque groupe étant éventuellement substitués par un ou plusieurs groupements choisis parmi halogène, alkyle (C₁-C₆) linéaire ou ramifié, alkoxy (C₁-C₆) linéaire ou ramifié,
- ----- représente une liaison simple ou une liaison double,
étant entendu que R₃ peut être greffé sur n'importe lequel des carbones du noyau indole/indoline le permettant,
leurs énantiomères et diastéréoisomères ainsi que leurs sels d'addition à un acide ou à une base pharmaceutiquement acceptable.

2. Composés de formule (I) selon la revendication 1 pour lesquels R₁, R₂ et R₃ représentent chacun un atome d'hydrogène, leurs énantiomères et diastéréoisomères ainsi que leurs sels d'addition à un acide ou à une base pharmaceutiquement acceptable.

3. Composés de formule (I) selon la revendication 1 pour lesquels Het est un groupe pyridinyle, pyrimidyle ou pipéridyle, leurs énantiomères et diastéréoisomères ainsi que leurs sels d'addition à un acide ou à une base pharmaceutiquement acceptable.

4. Composés de formule (I) selon la revendication 1 pour lesquels Het est un groupe pyridinyle, leurs énantiomères et diastéréoisomères ainsi que leurs sels d'addition à un acide ou à une base pharmaceutiquement acceptable.

5. Composés de formule (I) qui sont la *N*-(1*H*-indol-1-yl)-*N*'-(3-pyridinyl)urée et la *N*-(2,3-dihydro-1*H*-indol-1-yl)-*N*'-(3-pyridinyl)urée.

6. Procédé de préparation des composés de formule (I) **caractérisé en ce que** l'on utilise comme produit de départ un composé de formule (II): 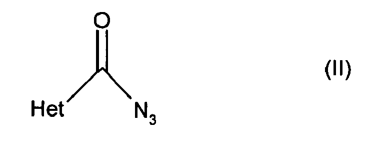 dans laquelle Het est tel que défini dans la formule (I),
composé (II), dont la décomposition thermique induit un dégagement de diazote, pour conduire à la formation d'un composé isocyanate de formule (III) que l'on peut isoler :
Het-N=C=O (III)
composé (III), qui est mis ensuite à réagir avec un composé de formule (IV) suivante : 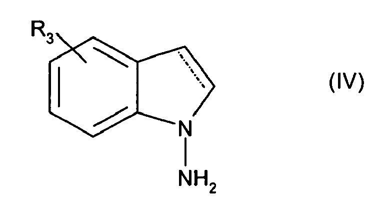 dans laquelle R₃ est tel que défini dans la formule (I),
pour conduire aux produits de l'invention de formule (I), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

7. Compositions pharmaceutiques contenant comme principe actif au moins un composé selon l'une quelconque des revendications 1 à 5, en combinaison avec un ou plusieurs excipients ou véhicules inertes, non toxiques, pharmaceutiquement acceptables.

8. Compositions pharmaceutiques selon la revendication 7 utiles dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.
